# EUROPEAN PATENT SPECIFICATION

(11) **EP 1 639 950 B1**
(45) Date of publication and mention of the grant of the patent: **24.06.2009**
(21) Application number: 05255954.9
(22) Date of filing: 26.09.2005
(51) Int. Cl.: A61B 17/17

(54) **Triangular handle surgical drill guide**
Chirurgische Bohrführung mit dreieckförmigem Griff
Guide de foret chirurgical avec une poignée triangulaire

(30) Priority: 27.09.2004 US 951107
(43) Date of publication of application: 29.03.2006
(73) Proprietor: DePuy Mitek, Inc., Raynham, MA 02767 (US)
(72) Inventor: Wenstrom, Richard F., Jr., Norwood, MA 02062 (US); Oren, Ran, 25130 (IL)
(74) Representative: Fisher, Adrian John

(56) References cited:
- US-A- 3 867 932
- US-A- 5 306 278
- US-A- 5 681 333
- US-A- 5 840 078
- US-A- 5 951 559
- US-A1- 2004 073 227

## Description

### FIELD

The field of art to which this invention relates is medical devices for use in surgical procedures, more specifically, arthroscopic drill guides.

### BACKGROUND

Medical devices and methods for attaching soft tissue to bone are known in the art. Of particular interest in orthopedic reconstructive surgery, in particular in sports medicine procedures, are suture anchors. A suture anchor is typically inserted into and fixed in a bore hole drilled into a bone at a surgical repair site. Sutures are typically attached to the anchor and are used to approximate the soft tissue to the bone in order to effect the repair. For many repair procedures, accuracy in the placement of suture anchors in bone is required to achieve consistently positive surgical outcomes, requiring substantial skill on the part of the orthopedic surgeon. Accurate placement of bore holes and suture anchors can be particularly challenging when an orthopedic repair is performed arthroscopically, as both access to and visibility of an arthroscopic surgical site may be more limited than is the case with open surgical procedures. For example, accurately drilling bore holes in the glenoid rim for placing suture anchors during an arthroscopic Bankart repair procedure can be difficult for even a very experienced surgeon.

With the increasing popularity of arthroscopic repairs such as shoulder rotator cuff repairs, capsulolabral reconstruction, and superior labral anterior to posterior (SLAP) lesion repair, as well as repairs in other body joints including the ankle, knee, elbow and foot, surgeons increasingly need to perform these procedures accurately and repeatably.

Accordingly, a significant need exists for novel devices and methods that provide for the accurate placement of suture anchors used in orthopedic surgical procedures.

US-A-2004/0073227 discloses an instrument having the precharacterising features of claim 1.

### SUMMARY

Therefore, as claimed in claim 1, a novel surgical instrument useful as an arthroscopic drill guide is disclosed. The instrument has an elongated tubular member having a distal end, a proximal end, a tubular wall, and a longitudinal axis. At least two tang members extend distally from the distal end. An elongated handle is connected to the tubular body proximal to the tangs. The handle has an external surface and a longitudinal axis. The handle has a substantially triangular external cross- section about its longitudinal axis. The surface contains at least one tactile reference mark. The tactile reference mark has a predetermined alignment with the tang members. A cannulated passage extends through the tubular member and handle. The passage has a proximal opening, a distal opening and a lumen in communication with said openings.

These and other aspects and advantages of the present invention will become more apparent from the following description and accompanying drawings.

### BRIEF DESCRIPTION OF THE DRAWINGS

FIG. 1 is a perspective view of an embodiment of a triangular handle drill guide of the present invention.
FIG. 2 illustrates the triangular drill guide of FIG. 1 rotated 120°.
FIG. 3 is an end view of the triangular handle drill guide of FIG. 1 taken along View Line 3-3, illustrating the handle and the lumen.
FIG. 4 illustrates the distal end of an embodiment of a dovetail triangular handle drill guide of the present invention.
FIG. 5 illustrates the distal end of an embodiment of a sawtooth triangular handle drill guide of the present invention.
FIG. 6 illustrates the distal end of an embodiment of a hybrid triangular handle drill guide of the present invention.
FIG. 7 illustrates the distal end of an embodiment of a fishmouth triangular handle drill guide of the present invention.
FIGS. 8a-d illustrate a soft tissue reattachment procedure using a drill guide of the present invention.

### DESCRIPTION

Certain exemplary embodiments will now be described to provide an overall understanding of the aspects and features of the methods, apparatus, and systems of use disclosed herein. Examples of these embodiments and features are illustrated in the drawings. Those of ordinary skill in the art will understand that the apparatus, systems and methods of use disclosed herein can be adapted and modified to provide apparatus, systems and methods for other applications and that other additions and modifications can be made without departing from the scope of the present disclosure. For example, the features illustrated or described as part of one embodiment or one drawing can be used on another embodiment or another drawing to yield yet another embodiment. Such modifications and variations are intended to be included within the scope of the present disclosure.

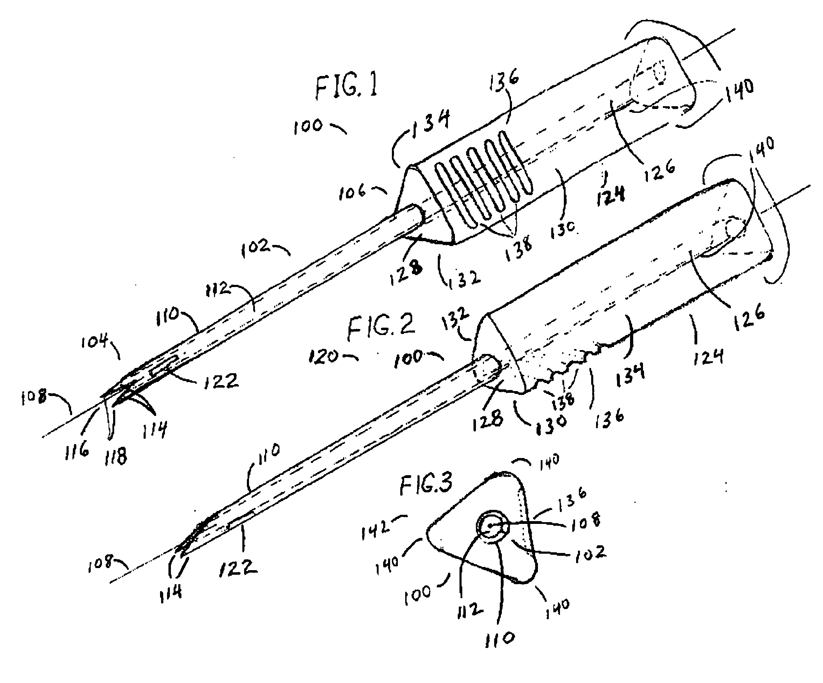

FIG. 1 illustrates an exemplary embodiment of a triangular handle drill guide 100 according to the present invention. The drill guide 100 is seen to have an elongated tubular body 102 having a distal end 104, a proximal end 106 and a longitudinal axis 108. The body also has a tubular wall 110 defining an axial body passage or lumen 112 along the longitudinal axis 108, that is, the body is cannulated. Preferably, the body 102 is substantially circular in cross-section, but may have other geometric cross-sections as well.

The tubular body 102 is seen to have two tang members or tangs 114 that extend distally from the distal end 104. The two tangs 114 are seen to be of equal length, but may have different lengths if desired. The two tangs 114 as shown define a V-shaped indentation 116 in the distal end 104. The two tangs 114 are seen to have sharp distal points 118, but if desired in an alternate embodiment, the two tangs 114 may have substantially rounded distal points. The two tangs 114 are adapted for straddling a bony prominence for positioning the drill guide 100 on a bone, for example a glenoid rim of a human shoulder. In other embodiments, the distal end 104 of the tubular body 102 includes one or more additional distal tangs or tang members to the two tangs 114. The tubular body 102 may be constructed from conventional biocompatible materials having adequate structural strength to support passing a drill through the axial body passage 112 for drilling in bone. Examples of suitable materials for construction of the body 102 include surgical steel, stainless steel, for example Type-302 stainless steel, aluminum, ceramics and plastics, etc. FIG. 2 illustrates a rotated view 120 of the drill guide 100, the view being rotated approximately 120 degrees about the axis 108 relative to the view of FIG. 1. As can be seen in FIG. 2, the two tang members 114 are asymmetrically (that is, not diametrically) positioned about the axis 108, in a "dovetail" configuration (that is, a dovetail-type drill guide). In another embodiment, the two tang members 114 are diametrically opposed about the axis 108.

The tubular body 102 may include one or more openings 122 through the wall 110 in or near distal end 104. The one or more openings 122 enable visualization of a medical device positioned within the axial body passage 112. , The medical device may be, for example, a bone drill or may be a conventional surgical obturator. The obturator is a blunt-ended instrument that can be positioned within the drill guide 100. In a surgical procedure using the drill guide 100, an obturator is positioned within the axial body passage 112 during positioning of the drill guide 100 on a bone. In addition the medical device may be a suture anchor, surgical drill, or other conventional surgical instrument. Preferably, the tubular body 102 has four or more openings 122, but may have fewer.

The drill guide 100 is also seen to have an elongated proximal handle 124 connected to the body 102 along the axis 108 proximal to the one or more openings 122. The handle 124 also includes an axial handle passage 126 aligned with the axial body passage 112. That is, the drill guide is fully cannulated along its longitudinal axis. The handle 124 is seen to have a substantially triangular cross-section 128 about the axis 108, a first face 130, a second face 132 and a third face 134. The first face 130, the second face 132 and the third face 134 are illustrated as substantially planar and rectangular in the embodiment of FIG. 1 and FIG. 2. In other embodiments, one or more of the first 130, the second 132 and the third face 134 may have another shape. The handle 124 is longitudinally tapered and each of the first 130, the second 132 and the third face 134 is substantially trapezoidal. In another embodiment, one or more of the first 130, the second 132 and the third face 134 is curved. The handle 124 may be constructed from the same material as the body 102 or from another conventional biocompatible material. For example, the body 102 may be constructed from surgical stainless steel and the handle 124 from a hard plastic such as polycarbonate. In another embodiment, the body 102 and the handle 124 are both constructed from a single type of material. In yet another embodiment, the drill guide 100 is fabricated as a unitary device from a single piece of material, i.e., either machined or molded.

The handle 124 has a predetermined, fixed rotational orientation about the axis 108 relative to the body 102. The handle 124 also includes at least one tactile reference mark, and is seen in FIG. 1 to include a plurality of reference marks 136, by which a user of the drill guide 102 may tactilely detect the rotational orientation of the drill guide 102 and thereby the rotational orientation of the two tangs 114. That is, the tactile reference marks 136 and the two tangs 114 have a predetermined alignment. The reference mark 136 may be any type of mark that can be sensed by touch while using the drill guide 102 and which is compatible with construction of a surgical instrument. Examples of types of tactile reference marks according to the present invention include raised areas, depressed areas or combinations thereof, and variations in surface texture, such as knurling. Tactile reference marks may be positioned on one or more of the first 130, the second 132 and the third face 134 of the handle 124. Tactile reference marks may also be positioned on one or more edge between adjacent surfaces on the handle 124.

The tactile reference mark 136 may include one or more grooves in a surface of the handle 124. In the embodiment illustrated in FIG. 1 and FIG. 2, the tactile reference mark 136 is seen to include a plurality of spaced-apart grooves 138 on the first face 130, for tactilely distinguishing the first face 130 from the second face 132 and the third face 134. Optionally, one or more visible markings on the handle 124 also distinguish the first face 130 from the second face 132 and the third face 134. In other embodiments, the e tactile reference mark 136 comprises tactile reference marks on more than one face of the handle 124, and the tactile reference marks may have different physical configurations to provide the user with a tactile distinguishment.

In the embodiment illustrated in FIG. 1 and FIG. 2, the plurality of grooves 138 tactilely identify the first face 130 as substantially aligned in a plane parallel to a plane that includes the two tangs 114 and is substantially parallel to the axis 108. Any predetermined alignment between the tactile reference mark 136 and the two tang members 114 may be selected for a drill guide of the present invention. In another embodiment, the two tangs 114 are positioned diametrically opposed about the axis 108 and the tactile reference mark 136 is aligned with one of the two tangs 114. In a further embodiment, a first one of the two tangs 114 is longer than a second one of the two tangs 114. In yet another embodiment, a drill guide of the present invention includes three or more tangs and a handle having a surface on which a tactile reference mark is aligned with one or more of the at least three tangs.

As also seen in FIG. 3, the handle is seen to include three longitudinal edges 140. The three longitudinal edges 140 are rounded (radiused). In other embodiments, one or more of the three edges 140 may have other shapes such as squared-off, beveled, or faceted. In still other embodiments, a tactile reference mark is positioned on one or more of the three edges 140. FIG. 3 illustrates an end view 142 of the drill guide 100 illustrated in FIG. 1 and FIG. 2. In FIG. 3, the tubular body 102 is seen to be circular in cross-section about the axis. The body 102 may also have other geometric cross-sections including polygonal, etc., In FIG. 3, the axial body passage 112 is seen to be circular in cross-section. In another embodiment, at least one of the axial body passage 112 and the axial handle passage 126 is polygonal in cross-section, or may have other geometric cross-sections.

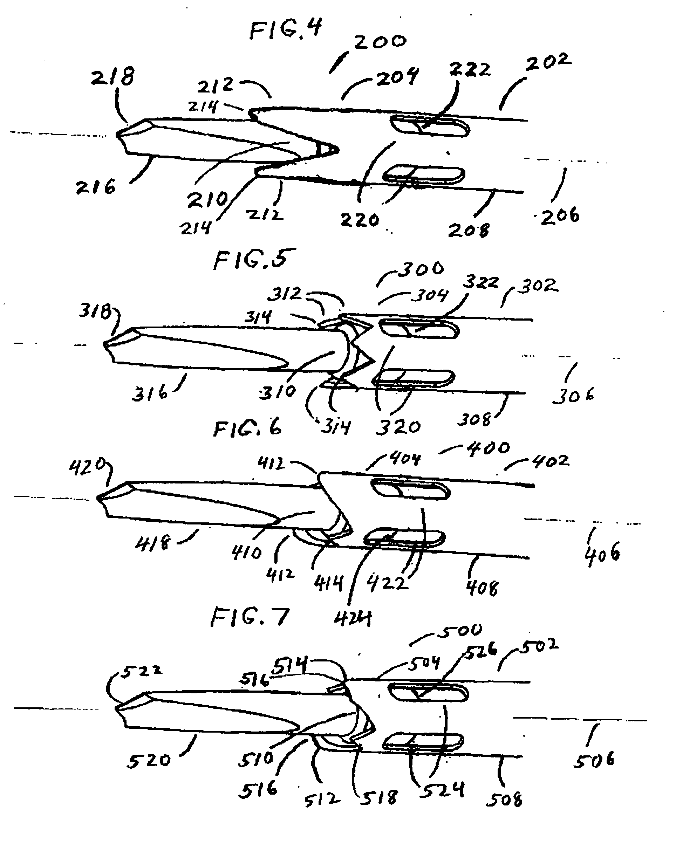

FIG. 4 illustrates distal end detail of another embodiment of a dovetail-type drill guide 200 of the present invention. The dovetail-type drill guide 200 is seen to include an elongated tubular body 202, a distal end 204, a longitudinal axis 206 and a tubular wall 208 defining a longitudinal passage 210. The dovetail-type drill guide 200 is also seen to include two tangs 212 extending distally from the distal end 204 of the body 202. The two tangs 212 are asymmetrically positioned about the axis 206. In an alternate embodiment, the two tangs 212 are positioned diametrically opposed about the axis 206. The two tangs 212 are seen to have pointed distal tips 214. In an alternate embodiment, the distal tips 214 of the two tangs 212 are rounded. A drill 216 is shown positioned within the longitudinal passage 210 and extending distally beyond the distal end 204 to a cutting tip 218. The body 202 may include one or more openings 220 in the wall 208 that enable visualization of the drill 216 within the passage 210. The drill 216 may include a visible mark 222 for indicating the longitudinal position of the drill 216 in the passage 210.

FIG. 5 illustrates distal end detail of an embodiment of a sawtooth-type drill guide 300 of the present invention. The sawtooth-type drill guide 300 is seen to include an elongated tubular body 302, a distal end 304, a longitudinal axis 306 and a tubular wall 308 defining a longitudinal passage 310. The sawtooth-type drill guide 300 is also seen to include a plurality of tangs 312 distributed circumferentially around and extending distally from the distal end 304. The plurality of tangs 312 may include any number of tangs. The plurality of tangs 312 typically includes between three and twenty tangs. As illustrated, the plurality of tangs 312 have pointed distal tips 314. Alternately, the distal tips 314 of the tangs 312 may be rounded or have other geometric configurations. A drill 316 is shown positioned within the longitudinal passage 310 and extending distally beyond the distal end 304 to a cutting tip 318. The body 302 may include one or more openings 320 in the wall 308 that enable visualization of the drill 316 within the passage 310. The drill 318 may include a visible mark 322 for indicating the longitudinal position of the drill 316 in the passage 310.

FIG. 6 illustrates distal end detail of an embodiment of a hybrid-type drill guide 400 of the present invention. The hybrid-type drill guide 400 is seen to include an elongated tubular body 402, a distal end 404, a longitudinal axis 406 and a tubular wall 408 defining a longitudinal passage 410. The hybrid-type drill guide 400 is also seen to include two primary tangs 412 extending distally from the distal end 404, and one or more secondary tangs 414 shorter than the two primary tangs 412. The one or more secondary tangs 414 also extend distally from the distal end and are positioned between the two primary tangs 412. In a surgical procedure according to the present invention, the one or more secondary tangs 414 are used along with the two primary tangs 412 to position the hybrid-type drill guide 400 on a bone. The two primary tangs 412 are seen to be asymmetrically positioned about the axis 406. In an alternate embodiment, the two primary tangs are positioned diametrically opposed about the axis 406. The two tangs are seen to have rounded distal tips 416. In an alternate embodiment, the two tangs have flattened distal tips. A drill 418 is shown positioned within the longitudinal passage 410 and extending distally beyond the distal end 404 to a cutting tip 420. The body 402 may include one or more openings 422 in the wall 408 that enable visualization of the drill 418 within the passage 410. The drill 418 is seen to include a visible mark 424 for indicating the longitudinal position of the drill 418 in the passage 410.

FIG. 7 illustrates distal end detail of an embodiment of a fishmouth-type drill guide 500 of the present invention. The fishmouth-type drill guide 500 is seen to include an elongated tubular body 502, a distal end 504, a longitudinal axis 506 and a tubular wall 508 defining a longitudinal passage 510. The fishmouth-type drill guide 500 includes a first tang 512 extending distally from the distal end 504 and a second tang 514 shorter than the first tang 512 also extending from the distal end 504. Each of the first tang 512 and the second tang 514 has a rounded distal tip 516. In an alternate embodiment, at least one of the first tang 512 and the second tang 514 has a flattened distal tip 516. The fishmouth-type drill guide 500 also includes one or more secondary tangs 518 shorter than the first tang 512 or the second tang 514. In a surgical procedure according to the present invention, the one or more secondary tangs 518 are used along with the first tang 512 and the second tang 514 to position the hybrid-type drill guide 400 on a bone. The second tang 514 is seen to be positioned diametrically opposed about the axis 506 from the first tang 512. A drill 520 is shown positioned within the longitudinal passage 510 and extending distally beyond the distal end 504 to a cutting tip 522. The body 502 may include one or more openings 524 in the wall 508 that enable visualization of the drill 520 within the passage 510. The drill 520 includes a visible mark 526 for indicating the longitudinal position of the drill 520 in the passage 510.

A drill used with a drill guide according to the present invention may be any conventional bone-penetrating device that can be positioned through a cannulation in the drill guide. The bone-penetrating devices include a fluted drill having one or more straight flutes, or having one or more spiral flutes. The bone penetrating device may also include spade-type drill, sharp bone-penetrating pointed instruments such as obturators, awls and the like and equivalents thereof

Any of the embodiments of drill guides above and equivalents thereof may be included in a surgical kit. Surgical kits simplify a surgeon's task of selecting surgical instruments for a surgical procedure, and assist in assuring that instruments selected by the surgeon work properly together. A surgical kit for an orthopedic repair surgery will include a drill guide according to the present invention and a drill sized for drilling bone through the drill guide. In a further embodiment the kit also includes an obturator sized for use with the drill guide. The obturator may optionally have a triangular cross-section handle. The surgical kit may also optionally include a suture anchor for use in a bone bore hole in bone prepared using the drill guide.

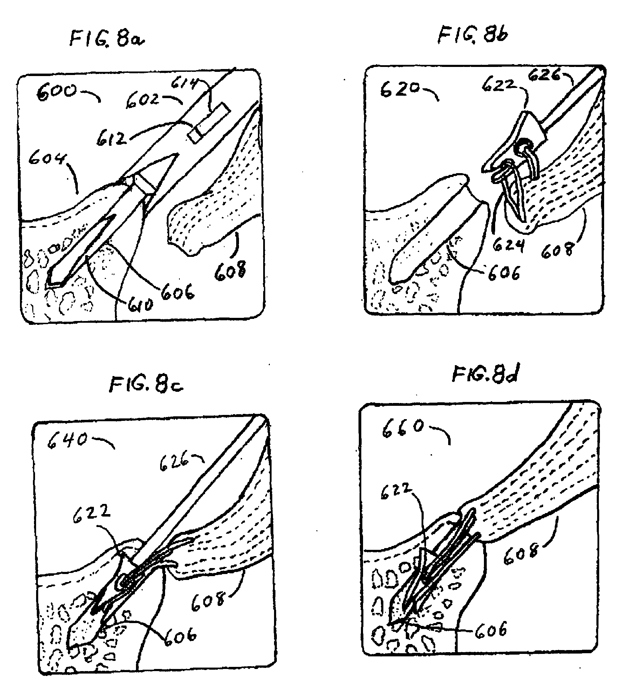

FIG. 8a through FIG. 8d illustrate an exemplary embodiment of an orthopedic surgical repair procedure using a drill guide of the present invention. FIG. 8a illustrates a drilling step 600 in which a dovetail-type drill guide 602 of the present invention is positioned straddling a portion of a bone 604 of a patient for accurate drilling of a bone bore hole 606 in preparation for reattaching detached soft tissue 608 to the bone 604. The drill guide 602 includes a substantially triangular cross-section handle (not shown) having at least one tactile reference mark according to the present invention. For the drilling step 600, a surgeon holds the drill guide by the handle and may use the at least one tactile reference mark to assist in orienting the drill guide 602 on the bone 604. For example, the surgeon may place a finger or thumb on the tactile reference mark for an enhanced tactile sense of the orientation of the drill guide 602 on the bone 604. A surgical drill 610 is shown penetrating the bone 604 to a depth that may be gauged using a visible reference mark 612 on the drill 610, the visible reference mark 612 being visible through an opening 614 in the drill guide 602.

FIG. 8b illustrates a soft tissue preparation step 620 in which a conventional suture anchor 622 has been attached to the soft tissue 608 using one or more lengths of suture 624. The suture anchor 622 is mounted to a suture anchor inserter 626 in preparation for insertion of the anchor 622 into the bone bore hole 606. Any type of conventional suture anchor, and equivalents thereof, may be used with the surgical procedures of the present invention in which drill guides of the present invention are used, including but not limited to threaded suture anchors, interference-type suture anchors, expandable anchors and toggle-type suture anchors, as well as tissue anchors that do not require the use of sutures. Tissue anchors and any sutures used in these procedures may be bioabsorbable or non-absorbable. As illustrated in FIGS. 8a-d, the suture anchor 622 is a bioabsorbable suture anchor that can be deployed in bone by a surgeon without the surgeon being required to tie a surgical knot at the operative site (a knotless anchor). FIG. 8c illustrates an insertion step 640, showing the suture anchor 622 inserted part way into the bore hole 606 using the inserter 626, drawing the soft tissue 608 toward the bone 604. FIG. 8d illustrates the completed repair 660. The anchor 622 has been fully inserted into and deployed in the bone bore hole 606, and the soft tissue 608 has been reapproximated to the bone 604.

Drill guides of the present invention may be used in surgical procedures in any part of the body including, but not limited to the shoulder, knee, ankle, foot, elbow and hand. Example surgical procedures in which drill guides of the present invention may be used include Bankart repair, SLAP lesion repair, acromioclavicular separation repair, rotator cuff repair, capsule shift and capsulolabral reconstruction, biceps tenodesis, deltoid repair, lateral and medial ankle instability, Achilles tendon repair and reconstruction, midfoot reconstruction, hallux valgus reconstruction, tennis elbow repair, biceps tendon reattachment, extra capsular knee repairs, patellar ligament and tendon avulsions, reattachment of: medial collateral ligament, lateral collateral ligament, posterior oblique ligament or joint capsule to tibia, and joint capsule closure to anterior proximal tibia.

Drill guides of the present invention may be of any size useful in surgery. In an exemplary embodiment, a drill guide of the present invention includes a 4.2 millimeter (mm) inner diameter axial body passage for use with a 2.9 mm diameter drill having an enlarged shank of nominally 4 mm diameter for passing through the longitudinal passage. In an embodiment, a longitudinal passage in a drill guide is adapted both for positioning a drill to drill a bore hole in bone, and for passing a suture anchor through the passage to the surgical site.

The drill guides of the present invention have several advantages including, but not limited to, advantages associated with the accuracy of bore hole positioning for orthopedic surgery, handling of the drill guides by a surgeon, and visualization of arthroscopic surgical sites.

As compared with known drill guides having handles that are substantially circular in cross section, or polygonal in cross section with a larger number of sides, a substantially triangular handle drill guide of the present invention provides a secure and angularly accurate grip for positioning a bore hole in bone, a particular advantage on a narrow prominence such as a glenoid rim in the shoulder. Tactile reference marks on the substantially triangular handle of a drill guide of the present invention also provide the surgeon with an accurate non-visual gauge of the orientation of distal tip features of the drill guide in procedures where visualization of a surgical site may be compromised, for example, for the positioning of distal tangs on a bone surface for drilling a bore hole during an arthroscopic orthopedic procedure. In addition, a substantially triangular handle of a drill guide or another surgical instrument of the present invention enhances the stability of the positioning of an instrument on a surgical tray or other surface without rolling, compared with instruments having substantially circular cross section handles or polygonal cross section handles having a larger number of sides.

Many changes in the details, materials, and arrangement of parts, herein described and illustrated, can be made by those skilled in the art. Although the invention has been shown and described with respect to detailed embodiments thereof, it will be understood by those skilled in the art that changes may be made without departing from the scope of the claimed invention. Accordingly, the following claims are not to be limited to the embodiments disclosed herein.

## Claims

1. A surgical instrument, comprising:
an elongated tubular member (102) having a distal end (104), a proximal end (106), a tubular wall (110), a longitudinal axis (108), and at least two tangs (114) extending distally from substantially the distal end;
an elongated handle (124) extending from the tubular member (102) proximal to the at least two tang members (114), and having an external surface; and,
a cannulated passage (112, 126) extending through the tubular member (102) and handle (124), said passage having a proximal opening, a distal opening and a lumen in communication with said openings;
two of the at least two tang members (114) are positioned asymmetrically or diametrically opposed about the axis (108); and
the external surface of the handle (124) has at least one tactile reference mark (136) on the surface, the at least one tactile reference mark having a predetermined alignment with the at least two tang members (114) ; **characterised in that**
the external surface of the handle (124) has a substantially triangular external cross-section about the axis (108).

2. The surgical instrument of claim 1 additionally comprising an opening (122) penetrating the wall (110) transverse to the axis (108).

3. The surgical instrument of claim 1 wherein one of the at least two tang members (114) is longer than another of the at least two tang members.

4. The surgical instrument of claim 1 wherein each of the at least two tang members (114) terminates in a distal point.

5. The surgical instrument of claim 1 wherein the tubular member (102) and the handle (124) comprise a unitary structure.

6. The surgical instrument of claim 1 wherein the at least one tactile reference mark (136) comprises a plurality of grooves.

7. The surgical instrument of claim 1, wherein the tubular member (102) has a circular cross-section.

8. The surgical instrument of claim 1, wherein the cannulated passage (112, 126) has a circular cross-section.

9. A surgical instrument according to claim 1, wherein the instrument is a surgical drill guide, the at least two tang members (114) are pointed and adapted for engagement with tissue, and the body (102) and the handle (124) are adapted for passing a surgical drill along the axis (108) from the proximal end to the distal end through the cannulated passage (112, 126).

10. The surgical drill guide of claim 9 wherein two of the at least two tang members (114) are asymmetrically positioned about the axis (108).

11. The drill guide of claim 10 wherein the at least two tang members comprises two tangs.

12. The drill guide of claim 9 wherein one of the at least two tang members (114) is longer than another of the at least two tang members.

13. The drill guide of claim 9 further comprising an opening (122) penetrating the tubular body (102) transverse to the axis (108).

14. The drill guide of claim 9 wherein the tubular body (102) and the handle (124) comprise a unitary structure.

## Patentansprüche

1. Chirurgisches Instrument, umfassend:
ein längliches rohrförmiges Element (102) mit einem distalen Ende (104), einem proximalen Ende (106), einer rohrförmiger Wand (110), einer Längsachse (108) und mindestens zwei Domen (114), die sich von im wesentlichen dem distalen Ende distal erstrecken;
einen länglichen Handgriff (124), der sich von dem rohrförmigen Element (102) proximal zu den mindestens zwei Domenelementen (114) erstreckt und eine Außenfläche aufweist; und
einen kannülierten Durchgang (112, 126), der sich durch das rohrförmige Element (102) und den Handgriff (124) erstreckt, wobei der Durchgang eine proximale Öffnung, eine distale Öffnung und ein Lumen in Verbindung mit den Öffnungen aufweist;
wobei zwei der mindestens zwei Dornenelemente (114) asymmetrisch oder diametral gegenüberliegend um die Achse (108) positioniert sind; und
die Außenfläche des Handgriffes (124) mindestens eine taktile Referenzmarkierung (136) auf der Fläche aufweist, wobei die mindestens eine taktile Referenzmarkierung eine vorab festgelegte Ausrichtung mit den mindestens zwei Domenelementen (114) aufweist;
**dadurch gekennzeichnet, dass** die Außenfläche des Handgriffes (124) einen im wesentlichen dreieckigen Außenquerschnitt um die Achse (108) aufweist.

2. Chirurgisches Instrument nach Anspruch 1, zusätzlich umfassend eine Öffnung (122), die die Wand (110) quer zur Achse (108) durchdringt.

3. Chirurgisches Instrument nach Anspruch 1, **dadurch gekennzeichnet, dass** eines der mindestens zwei Dornenelemente (114) länger als das andere der mindestens zwei Dornenelemente ist.

4. Chirurgisches Instrument nach Anspruch 1, **dadurch gekennzeichnet, dass** jedes der mindestens zwei Dornenelemente (114) in einer distalen Spitze endet.

5. Chirurgisches Instrument nach Anspruch 1, **dadurch gekennzeichnet, dass** das rohrförmige Element (102) und der Handgriff (124) eine einheitliche Struktur aufweisen.

6. Chirurgisches Instrument nach Anspruch 1, **dadurch gekennzeichnet, dass** die mindestens eine taktile Referenzmarkierung (136) eine Vielzahl von Nuten aufweist.

7. Chirurgisches Instrument nach Anspruch 1, **dadurch gekennzeichnet, dass** das rohrförmige Element (102) einen kreisförmigen Querschnitt aufweist.

8. Chirurgisches Instrument nach Anspruch 1, **dadurch gekennzeichnet, dass** der kannülierte Durchgang (112, 126) einen kreisförmigen Querschnitt aufweist.

9. Chirurgisches Instrument nach Anspruch 1, **dadurch gekennzeichnet, dass** das Instrument eine chirurgische Bohrführung ist, die mindestens zwei Dornenelemente (114) spitz sind und für einen Eingriff mit Gewebe gestaltet sind und der Körper (102) und der Handgriff (124) zum Hindurchlassen eines chirurgischen Bohrers entlang der Achse (108) von dem proximalen Ende zum distalen Ende durch den kannülierten Durchgang (112, 126) gestaltet sind.

10. Chirurgische Bohrführung nach Anspruch 9, **dadurch gekennzeichnet, dass** zwei der mindestens zwei Dornenelemente (114) um die Achse (108) asymmetrisch positioniert sind.

11. Bohrführung nach Anspruch 10, **dadurch gekennzeichnet, dass** die mindestens zwei Dornenelemente zwei Domen aufweisen.

12. Bohrführung nach Anspruch 9, **dadurch gekennzeichnet, dass** eines der mindestens zwei Dornenelemente (114) länger als das andere der mindestens zwei Dornenelemente ist.

13. Bohrführung nach Anspruch 9, ferner umfassend eine Öffnung (122), die den rohrförmigen Körper (102) quer zur Achse (108) durchdringt.

14. Bohrführung nach Anspruch 9, **dadurch gekennzeichnet, dass** der rohrförmige Körper (102) und der Handgriff (124) eine einheitliche Struktur aufweisen.

## Revendications

1. Instrument chirurgical, comprenant :
■ un élément tubulaire allongé (102) qui présente une extrémité distale (104), une extrémité proximale (106), une paroi tubulaire (110), un axe longitudinal (108), et au moins deux tenons (114) qui s'étendent de manière distale sensiblement à partir de l'extrémité distale ;
■ une poignée allongée (124) qui s'étend à partir de l'élément tubulaire (102) de manière proximale par rapport aux deux éléments de tenons (114) au moins, et qui présente une surface extérieure ; et,
■ un passage en forme de canule (112, 126) qui s'étend à travers l'élément tubulaire (102) et la poignée (124), ledit passage présentant une ouverture proximale, une ouverture distale et une lumière en communication avec lesdites ouvertures ;
■ deux des au moins deux éléments de tenons (114) sont positionnés de manière asymétrique ou diamétralement opposés autour de l'axe (108) ; et,
■ la surface extérieure de la poignée (124) présente au moins une marque de référence tactile (136) sur la surface, l'au moins une marque de référence tactile présentant un alignement prédéterminé avec les au moins deux éléments de tenons (114) ;
**caractérisé en ce que** la surface extérieure de la poignée (124) présente une section transversale extérieure sensiblement triangulaire autour de l'axe (108).

2. Instrument chirurgical selon la revendication 1, comprenant en outre une ouverture (122) qui pénètre dans la paroi (110) de manière transversale par rapport à l'axe (108).

3. Instrument chirurgical selon la revendication 1, dans lequel l'un des au moins deux éléments de tenons (114) est plus long que l'autre des au moins deux éléments de tenons.

4. Instrument chirurgical selon la revendication 1, dans lequel chacun des au moins deux éléments de tenons (114) se termine en un point distal.

5. Instrument chirurgical selon la revendication 1, dans lequel l'élément tubulaire (102) et la poignée (124) constituent une structure unitaire.

6. Instrument chirurgical selon la revendication 1, dans lequel l'au moins une marque de référence tactile (136) comprend une pluralité de rainures.

7. Instrument chirurgical selon la revendication 1, dans lequel l'élément tubulaire (102) présente une section transversale circulaire.

8. Instrument chirurgical selon la revendication 1, dans lequel le passage en forme de canule (112, 126) présente une section transversale circulaire.

9. Instrument chirurgical selon la revendication 1, dans lequel l'instrument est un guide de foret chirurgical, les au moins deux éléments de tenons (114) sont pointus et aptes à venir en prise avec le tissu, et le corps (102) et la poignée (124) sont aptes à passer un foret chirurgical le long de l'axe (108) à partir de l'extrémité proximale vers l'extrémité distale à travers le passage en forme de canule (112, 126).

10. Guide de foret chirurgical selon la revendication 9, dans lequel deux des au moins deux éléments de tenons (114) sont positionnés de manière asymétrique autour de l'axe (108).

11. Guide de foret selon la revendication 10, dans lequel les au moins deux éléments de tenons comprennent deux tenons.

12. Guide de foret selon la revendication 9, dans lequel l'un des au moins deux éléments de tenons (114) est plus long que l'autre des au moins deux éléments de tenons.

13. Guide de foret selon la revendication 9, comprenant en outre une ouverture (122) qui pénètre dans le corps tubulaire (102) de manière transversale par rapport à l'axe (108).

14. Guide de foret selon la revendication 9, dans lequel le corps tubulaire (102) et la poignée (124) constituent une structure unitaire.
